(19) Europäisches Patentamt

European Patent Office

Office européen des brevets

(11) **EP 0 718 306 B1**

(12) **EUROPEAN PATENT SPECIFICATION**

(45) Date of publication and mention
of the grant of the patent:
**30.05.2001 Bulletin 2001/22**

(51) Int Cl.⁷: **C07K 5/06**

(21) Application number: **95203533.5**

(22) Date of filing: **18.12.1995**

(54) **Crystallization of alpha-L-aspartyl-L-phenylalanine methyl ester from aqueous solution**

Crystallisierung vom Alpha-L-Aspartyl-L-phenylalanylmethylester aus wässrigen Lösungen

Crystallisation de l'ester méthylique de alpha-L-aspartyl-L-phénylalanine à partir de solution aqueuses

(84) Designated Contracting States:
**AT BE CH DE DK ES FR GB GR IE IT LI LU MC NL PT SE**

(30) Priority: **22.12.1994 EP 94203726**

(43) Date of publication of application:
**26.06.1996 Bulletin 1996/26**

(73) Proprietor: **HOLLAND SWEETENER COMPANY V.O.F.**
**6212 XW Maastricht (NL)**

(72) Inventors:
• **Rijkers, Marinus P.W.M.**
 **NL-6181 DH Stein (NL)**
• **Toussaint, Antoon G.T.**
 **NL-6416 GX Heerlen (NL)**
• **Vrinzen, Alexander P.M.**
 **NL-6231 GZ Meerssen (NL)**

(56) References cited:
**EP-A- 0 272 062**

**Description**

BACKGROUND OF THE INVENTION

Field of the invention

The invention relates to a method for cooling an aqueous solution of $\alpha$-L-aspartyl-L-phenylalanine methyl ester (hereinafter also referred to as aspartame or "APM") and crystallization of $\alpha$-L-aspartyl-L-phenylalanine methyl ester therefrom with avoiding of turbulence in the aqueous crystallizing system.

Description of the related art

Aspartame, the $\alpha$7-dipeptide ester L-aspartyl-L-phenylalanine methyl ester ("APM"), is an important synthetic low-calorie sweetening agent which is about 200 x as sweet as sugar and has an exceptionally good taste pattern without, for instance, a bitter aftertaste. The sweetener is used as such in a wide range of products such as soft drinks, sweets, tabletop sweeteners, pharmaceutical products, and the like.

Aspartame can be prepared by various known routes. There exist, for instance, routes whereby N-protected L-aspartic acid or the anhydride thereof and (L-)phenylalanine or the methyl ester thereof are chemically coupled, the protecting group optionally being removed later and APM being obtained by esterification if still necessary. Examples of such processes are disclosed in, for instance, US-A-3,786,039. In processes for chemical coupling of aspartame usually relatively large amounts of $\beta$-APM are formed as a side-product, and work-up of the desired $\alpha$-APM often occurs through formation of e.g. the APM.HCl-salt and neutralization crystallization. Such methods inevitably lead to production of large amounts of inorganic salt.

There also exist enzymatic processes for the production of APM whereby, for instance, N-protected L-aspartic acid and (DL-)-phenylalanine methyl ester are selectively coupled to form the LL-$\alpha$-dipeptide derivative and are subsequently converted to APM. Such a process is described in, for instance, US-A-4,116,768.

In any APM production process one of the final process steps is to obtain APM in crystalline form from the solvent in which it is present and wherein also other reaction (by-)products and/or decomposition products occur. Usually the solvent is an aqueous solvent, that is water or a mixed solvent of water and up to about 25% (wt.) of a water-miscible organic solvent, especially a lower alcohol of one to three carbon atoms. As used herein, the term "aqueous" therefore specifically will mean "water, or water containing up to about 25% (wt.) of a $C_{1-3}$ alcohol.

The meaning of the term "APM" as used herein for the starting material does not include physiologically acceptable salts of APM such a the hydrochloric acid salt thereof ("APM.HCl"), but may include APM obtained after neutralization of such salts.

A method for crystallizing APM from aqueous solutions is described in EP-A-91787 (hereinafter also referred to as '787). According to that method APM is crystallized, at least for the major part of crystallization, using conductive heat transfer for cooling without effecting forced flow, i.e. under conditions where turbulence in the aqueous system is avoided. This method requires special equipment and leads to the formation of a hard, sherbet-like, pseudo-solid phase. Due to the required method of cooling, which will not enable a heat transfer coefficient above at best 100 W/ $m^2$.K on average during the cooling process, the cooling time is relatively long. Moreover it has been found that APM crystals obtained by the method of '787 are difficult to handle in wet granulating processes. These are substantial disadvantages of this prior art method.

Accordingly, it is an object of the present invention to provide a rapid method for crystallizing APM by cooling from its aqueous solutions which moreover results in crystals suitable for being treated in a wet granulating process and still having acceptable other crystal properties.

Summary of the invention

This object is achieved in that the method for cooling an aqueous solution of $\alpha$-L-aspartyl-L-phenylalanine methyl ester and crystallization of a-L-aspartyl-L-phenylalanine methyl ester therefrom with avoiding of turbulence in the aqueous crystallizing system is performed by (i) feeding a hot aqueous solution of $\alpha$-L-aspartyl-L-phenylalanine methyl ester to means for dispersing droplets of said solution; and (ii) dispersing said droplets into a water-immiscible liquid having a temperature at least about 20°C lower than said hot solution, and having such a temperature difference with the hot solution that no nucleation will occur in the droplets while these are passing the water-immiscible liquid, the water-immiscible liquid having a density smaller than the density of the aqueous solution at the same temperature, and having a viscosity of less than 10 mPa.s; and (iii) cooling said water-immiscible liquid so that said droplets are effectively cooled to reach an initial relative supersaturation of $\alpha$-L-aspartyl-L-phenylalanine methyl ester within the droplet in the range of 1 to 6, preferably 1.2 to 4; and (iv) collecting the cooled droplets after having passed the water-immiscible liquid to crystallize α-L-aspartyl-L-phenylalanine methyl ester without any mechanical agitation; (v) and providing sufficient time for crystallizing α-L-aspartyl-L-phenylalanine methyl ester from the collected aqueous phase.

This method provides an elegant and rapid way for crystallizing APM by cooling from its aqueous solutions, and results in crystals suitable for being treated in a wet granulating process while still having acceptable other crystal properties. In addition to aspartame, other products that show good crystallization behaviour at high supersaturation may also be crystallized according to the present invention. Preferably, however, aspartame is crystallized.

In addition it has turned out that the present method, when the crystalline product obtained is dried, leads to aspartame particles having a smooth surface, whereas the product obtained by the state of the art static crystallization has a rough surface; the smooth surface is especially preferred in case moistening of the product will be important, such as for instance in wet granulating. It is noticed that the dissolution rate of the crystals obtained according to the present invention may be slightly worse than that of crystals obtained according to the '787 method, but it is well known in the art that such dissolution rate may be improved by further treatment of the crystals, for instance by reducing average size, or by adding additives such as dissolution rate enhancers.

The present invention therefore relates to a method for cooling an aqueous solution of APM and crystallization of APM therefrom with avoiding of turbulence in the aqueous crystallizing system, which comprises (i) feeding a hot aqueous solution of α-L-aspartyl-L-phenylalanine methyl ester to means for dispersing droplets of said solution; and (ii) dispersing said droplets into a water-immiscible liquid having a temperature at least about 20°C lower than said hot solution, and having a temperature difference with the hot solution at which no nucleation will occur in the droplets while these are passing the water-immiscible liquid, the water-immiscible liquid having a density smaller than the density of the aqueous solution at the same temperature, and having a viscosity of less than 10 mPa.s; and (iii) cooling said water-immiscible liquid so that said droplets are effectively cooled to reach an initial relative supersaturation of α-L-aspartyl-L-phenylalanine methyl ester within the droplet in the range of 1 to 6, preferably 1.2 to 4; and (iv) collecting the cooled droplets after having passed the water-immiscible liquid to crystallize α-L-aspartyl-L-phenylalanine methyl ester without any mechanical agitation; (v) and providing sufficient time for crystallizing α-L-aspartyl-L-phenylalanine methyl ester from the collected aqueous phase.

Relative supersaturation as used herein relates to the metastable situation before the start of crystallization, and is defined by the ratio (σ) between a) the difference (Δc) in actually dissolved concentration of APM before crystallization starts and the saturated concentration of APM (c*) in the mother liquor at crystallization temperature, and b) said saturated concentration of APM in the mother liquor (c*). Thus, $\sigma = \Delta c / c^*$. The value of α before crystallization starts is the initial relative supersaturation.

c*, as used in this application, is calculated (for solutions of APM in pure water in the temperature range of the slurries obtained in the present method) according to the following formula:

$$c^* = 0.436 \cdot 10^{0.017 \cdot T} \text{ (in wt.\%)},$$

wherein T is temperature in °C. In the relevant temperature range this formula is in good agreement with the formula presented by Kishimoto et al. in "J.Chem.Tech.Biotechnol.", 1988, 43, pages 71-82, which reads:

$$c^* = 4.36 \cdot 10^{0.017 \cdot T} \text{ (in g/l; T in °C)}.$$

The latter formula (in g/l), however, is a bit more ambiguous.

As explained above the term aqueous solvent refers to water or to a mixed solvent of water and up to about 25% (wt.) of a water-miscible organic solvent, such as for instance a $C_{1-3}$ alcohol. Moreover, presence of a lower alcohol may be advantageous in further process steps, such as in further treatment of the slurry, for instance by solvent removal under vacuum. The main advantage of using mixed solvents, however, is that higher concentrations of (dissolved) APM can be achieved in the starting solution. The water-immiscible liquid used in general will be a poor solvent for APM, and at most only minor amounts of water will be soluble therein.

It is noticed here that the density of the aqueous solution of APM is dependent on the composition of that solution. Presence of a lower alcohol has a tendency of lowering the density. The density of the aqueous APM solution (measured at the temperature of the water-immiscible liquid) thus in general will be in the range of 0.95-1.00 g/cm³.

The temperature difference between the hot aqueous APM solution and the (colder) water-immiscible organic liquid should be substantial, e.g. at least about 20°C, in order that an acceptable yield of crystals is obtained in the lower layer (i.e. below the water-immiscible liquid). However, the temperature difference should not be so large that an excessive (burst of) nucleation would occur in the droplets while these are passing the water-immiscible liquid. This would lead to the undesired formation of small crystals.

The droplets falling into the (lower temperature) water-immiscible liquid will be cooled while passing through said liquid. This cooling takes place most efficiently, and can be calculated to occur with an overall heat transfer coefficient in the range of about 800 to about 3.000 $W/m^2.K$, in contrast to the heat transfer coefficient according to the conductive heat transfer cooling method of the state of the art, which amounts to about at best 100 $W/m^2.K$.

It is to be noticed that another method for quickly cooling and crystallizing APM from aqueous solutions has been described in EP-A-0523813, where such cooling is achieved by direct contact with ice. However, said method is clearly disadvantageous because on the one hand cooling is not equally efficient throughout the whole APM solution, and on the other hand extensive dilution of the original APM solution occurs due to the melting of the large amounts of ice needed.

In a preferred embodiment of the invention the means for dispersing droplets, for instance chosen from various types of nozzles (such as spray nozzles, pressure nozzles, rotating nozzles, spinning atomizers, gas-atomizing nozzles, vibrating nozzles, or impact nozzles), perforated - optinally vibrating - plates or tubes, droppers and orifices, or other means for forming droplets, are placed near the upper liquid level of the water-immiscible liquid; means for forming and dispersing droplets in general are relatively simple, inexpensive and commercially available, and the skilled man will easily be able to determine which type(s) will be best suited for the purpose. Preferably such type of dispersing means is chosen which gives quite uniform droplets, or so-called monodisperse droplets. The size of droplets to be formed may be adjusted in a wide range by the choice of dispersing means. Preferably the average range of droplet size (diameter) will be chosen narrowly.

Most preferred the means for dispersing droplets are placed at a short distance above the upper liquid level of the water-immiscible liquid, without being in direct contact with said liquid. In such way it is avoided that crystallization and/or clogging occurs at the outlet of the nozzle (or the like) which might lead to undesired interruptions of the process. The distance of the dispersing means to the upper surface of the water-immiscible liquid is not critical, but usually will not be more than 2 meters to avoid useless "dead volumes" in the equipment.

As the level of the water-immiscible liquid in the equipment may rise during operation of the method of the invention (and this certainly occurs during batchwise operation, when no withdrawal of slurry takes place from the bottom part of the crystallizing equipment), it is preferred that the means for dispersing can be moved vertically, and are being adjusted in height during the process according to the position of the upper liquid level. Such vertical adjustability of the dispersing means is less important or even superfluous in case the process of the invention is operated in a continuous mode, that is by (semi)- continuously removing the crystal slurry formed in the lower part of the equipment while feeding fresh aqueous APM solution at the top.

In order to ensure that the droplets formed in the dispersing means will have sufficient cooling time while passing the water-immiscible liquid, it is preferred that the dispersing takes place at least 10 cm above the lower level of the water-immiscible liquid. The minimum distance will be dependent on the droplet size.

For achieving good results the temperature of the hot aqueous solution of APM preferably should be at least 50°C and the concentration of APM therein at least 2.5 % by weight. If the temperature of the hot aqueous solution is lower than 50°C the crystallization yield will become too low; also, if concentration of APM therein is too low, the relative supersaturation which can be achieved in the process of the invention also will be relatively low, and unfavorable results will be obtained. As stated above, the temperature difference between the hot aqueous APM solution and the (colder) water-immiscible organic liquid should be at least about 20°C, and such that no nucleation would occur in the droplets while these are passing the water-immiscible liquid.

The droplets falling into the (lower temperature) water-immiscible liquid will be cooled while passing through said liquid. This cooling takes place most efficiently, and can be calculated to occur with an overall heat transfer coefficient in the range of about 800 to about 3.000 $W/m^2.K$.

Advantageously the lower temperature of the water-immiscible liquid (as compared to the hot aqueous solution of APM) is maintained at about constant level by cooling; preferably such cooling is done by indirect cooling. Indirect cooling as used here is meant to refer to either cooling at the surface area of the part of the crystallizing vessel/tube containing the water-immiscible liquid, or to cooling by externally circulating the water-immiscible liquid through a heatexchanger; the latter is preferably done by counter-currently feeding (and removing) the water-immiscible liquid, in such way that no aqueous phase is entrained to the external heat exchanger, to (and from) the crystallization vessel. This can be easily achieved by adequate design of the equipment, for instance by application of tangential feed lines and an overflow duct.

The temperature of the coolant used for cooling the water-immiscible liquid usually will be between -10 and +20°C. The skilled man easily will be able to determine optimum conditions depending of equipment used, temperature of the hot aqueous APM solution and water-immiscible liquid used.

Therefore, in case the water-immiscible liquid is circulated, and externally cooled, this is preferably done by withdrawing it from the top of the water-immiscible liquid layer, without entrainment of aqueous phase, and feeding it counter-currently near the lower level of said liquid. It is advantageous to carry out such withdrawal and feeding in such way that the more or less "stationary" conditions in the crystallizing equipment are least disturbed; this can be achieved, for instance, by use of baffles, tangential feeding, etc.

In a preferred embodiment of the present invention the water-immiscible liquid is contained in (one or more) tubular column(s), (each) being equipped with droplet dispersing means.

The dispersion of droplets should preferably occur at such a distance from the internal side-walls of the crystallizing equipment, especially in case (a) tubular column(s) is (/are) used, that the droplets formed in the dispersing means will not come into contact with said side-walls while moving down. Such contacts with the side-walls might lead to long residence times and undesired crystallization and scaling at those walls. The skilled man easily will be able to determine the optimum configuration and design of the equipment.

The diameter of the droplets of APM solution formed in the dispersion means may be chosen quite arbitrarily, though it is preferred that the diameter is in the range of 0.05 to 5 mm, most preferably 0.1 to 3 mm, on average. Best results are obtained if droplets are monodisperse, i.e. having quite uniform diameter. The diameter of the droplets is one of the factors influencing the cooling rate and the required residence time of the droplet in the water-immiscible liquid. As the diameter of the droplet is smaller, the cooling will be faster. Good crystal properties are obtained when droplets of 0.1 to 3 mm are being dispersed. The skilled man easily will be able to determine the optimum combination of dispersion means, droplet size, water-immiscible liquid and equipment.

The water-immiscible liquid may be chosen from a wide range of liquids having a low or negligible solubility in water and a specific density which is significantly lower than the density of the aqueous solution at the same temperature. Preferably it is chosen from the group of aliphatic and/or aromatic hydrocarbons having from 5 to 12 carbon atoms, and most preferably it is chosen to be toluene or n-heptane. The former, having a density being more close to that of water, has the advantage that the aqueous droplets falling into the liquid are dampened more effectively, so that their impact when reaching the lower aqueous crystallizing slurry is lowest and crystals formed or present in the lower part of the equipment are least affected. The latter has the advantage that even less entrainment into the aqueous system occurs, and that the crystallizing slurry is even more free of the organic liquid component than in case of e.g. toluene. In general, however, it can be found that the entrainment of water-immiscible liquid into the crystallizing slurry will be lower if impact of the droplets onto the crystal bed is lower. Such impact is, if the layer of water-immiscible liquid is not too thin, quite independent of height of that layer as the droplets move through the layer at about linear speed (terminal settling velocity). Measuring the terminal settling velocity of the droplets offers a good opportunity for determining the droplet diameter, since the terminal settling velocity is related to said diameter according to Stokes' law.

Preferably, the droplets passing the water-immiscible liquid are cooled from a temperature of 50°C or higher to a temperature in the range of 35 to 20°C, or lower. In case the aqueous solution also contains a lower alcohol, the APM concentration may be higher, and the start of nucleation will occur at a different relative supersaturation from that when using water as the sole solvent for APM under otherwise identical conditions.

The aqueous phase, after having passed the water-immiscible liquid, is collected in the bottom part of the crystallization equipment, preferably in a condition of low or negligible agitation or mechanical disturbance, for allowing crystallization to take place. Optionally the collected aqueous phase may be cooled further by indirect cooling.

It is preferred that the average residence time of the aqueous phase collected in the bottom part of the equipment, while being kept without mechanical agitation or the like, is at least 30 minutes. During said residence time a kind of network of APM crystals is being formed, which may be stronger or weaker depending on the amount of aspartame being present, but which if necessary can be easily destroyed by mechanical treatment. In any event the network formed according to this method is different from a so-called sherbet-like pseudo solid phase as is formed in static crystallization: the latter, when formed in a beaker, will retain all of the aqueous solvent when the beaker is turned upside-down.

The (lower part of the) network of APM formed in the present method thus can be removed as a slurry without influencing the upper part of the network, for instance, (continuously or batchwise) by using a mechanical screw-feeder. Continuous operation is most preferred.

The process of the present invention may be operated batchwise or in a (semi-)continuous mode. The latter modes are preferred in view of industrial feasibility of the process, and can be carried out advantageously by continuously withdrawing amounts of APM crystal slurry from the bottom part of the collected aqueous phase, either continuously or intermittently, after having remained there without mechanical agitation for at least 30 minutes on average.

As indicated above, optionally the collected aqueous phase may be cooled further by indirect cooling. By such further cooling the yield of crystals obtained can be increased. This may be done either by further cooling the bottom part of the crystallizing equipment, or by withdrawing crystal slurry therefrom to another vessel, cooled to a lower temperature in the range of 0-20°C, and carrying out the further crystallization in said additional vessel. Preferably the further crystallization then is done while stirring because this increases the cooling rate and crystal output per unit of time. Remarkably, this higher yield of crystals is obtained without negatively influencing crystal size and properties.

After crystallization of APM, optionally with further cooling, the collected aqueous phase and the water-immiscible liquid are separated, and any water-immiscible liquid remaining in the aqueous phase may be removed from it by a method known per se, such as evaporation.

The method of the invention can be carried out easily in equipment as shown schematically in the attached drawing (Figure 1). In this drawing, representing the situation as in the preferred mode of continuous operation, the numbers shown have the following meaning:

(1) represents the feedline through which the hot aqueous APM solution, which is prepared and kept at the elevated temperature in a storage vessel (not shown), is fed from the top into a vessel or column (2), which is partially filled with the water-immiscible liquid. Said feeding occurs through a dosage system (3), consisting of means for dispersing droplets. The dosage system may be adjusted vertically according to the upper level of the water-immiscible liquid in the vessel. (4) represents the zone of water-immiscible liquid in vessel (2), whereas (5) represents the slurry zone where the crystallization takes place. This zone optionally may be cooled by conductive heat transfer (5a). The water-immiscible liquid may be circulated through an external cooler (6) for being kept at about constant temperature while feeding the hot aqueous APM solution. Entrainment of aqueous phase to the external cooler (6), which is operated with a refrigerant (7), is avoided by appropriate positioning of the circulation lines and design of vessel (2), e.g. by installing an overflow duct. At the bottom part of vessel (2) means (8) are provided for discharging the slurry formed and transporting it to another vessel (9), equipped with a stirrer (10) where optional further cooling may take place. The crystal slurry finally obtained is discharged through outlet (11) to the further process steps for recovering and drying the APM crystals.

The present invention therefore also relates to such equipment as schematically shown in Figure 1. However, the equipment by no means is restricted to apparatus as shown in said Figure. The skilled man easily will be able to construct equipment according to the present invention; it is noticed here that this equipment very closely resembles "extraction equipment" and therefore demonstrates features of a dedicated extraction column.

The invention is now further explained by means of the following experiments, shown below as examples and comparative examples, without however being restricted thereto by any means.

Almost all experiments were performed using glass equipment consisting of a tubular column (height 75 cm; diameter 10 cm), having its middle part equipped with a jacket through which coolant could be circulated, and above which column a one liter feed vessel, containing aqueous APM solution at elevated temperature, was placed. The lower part of the column was provided with a discharge valve, through which the slurries finally obtained could be discharged. In the experiments the starting solution was fed into the column dropwise either through a 0.9 mm syringe (for obtaining droplets of about 3 mm in average diameter), or was pumped into the column through a dedicated nozzle (for obtaining droplets of < 0.5 mm in average diameter). The droplet diameter was determined, for each type of experiment and hot solution used, either by counting droplets and weighing, or by measuring the terminal settling velocity (Stokes' law). At the start of each experiment the column was filled with about 4 liter of water-immiscible liquid at a temperature level about the same as that of the slurry temperature aimed at without additional cooling of the slurry zone or slurry, and the experiment was continued until about 15 cm height (i.e. a volume of about 1 liter) of aqueous slurry was obtained in the bottom part of the column. However, it should be noticed that the effective time for the experiments is determined by the time the falling droplets are passing through the water-immiscible liquid. Further residence times in the bottom part are not critical and are merely intended for ensuring completion of crystallization.

During the experiments the temperature gradient of the water-immiscible liquid was kept at a minimum, as indicated, by cooling the middle part of the water-immiscible liquid zone by circulating a coolant of appropriate temperature through the jacket. The temperature of the slurry obtained was continuously monitored by a thermometer placed in the bottom part of the column. It also was established that this slurry temperature was only slightly higher (at most 4°C) than the temperature of the lower part of the water-immiscible liquid. Properties of the crystal slurries obtained were determined at the temperature of the slurry, after separating the slurry layer from the water-immiscible liquid layer, by determining the specific cake resistance at constant pressure difference, as described e.g. in EP-A-0399605.

For purposes of better comparability all specific cake resistance values are shown (as determined or re-calculated) at a pressure difference $\Delta P$ of 0.25 bar (25 kPa). For some of the slurries (of experiments 1, 2 and 3) also cake compressibility was determined (respectively being 0.56, 0.50 and 0.64, i.e. on average 0.57); this was done, according to standard methods, after determining the specific cake resistance at 5 chosen pressure differences.

Relevant details of the experiments and comparative experiments performed, with experimental conditions and results, are summarized in the following table 1.

TABLE 1

| Expt / Comp. Expt (C) with T/H[1) | Feed Compsn[2)]Wt% APM [Temp] (°C) | Slurry Temp. (°C) | concentration values (wt. %) and σ | | Droplet Size (mm) | Specfc Cake Rsstce (m/kg *10[9])[3)] |
|---|---|---|---|---|---|---|
| | | | c* σ | Δc | | |
| 1 T | 4 [65] | 23.5 | 1.09 2.67 | 2.91 | 0.1-0.5 | 1.9 |
| 2 H | 4 [65] | 22 | 1.03 2.88 | 2.97 | 0.1-0.5 | 2.3 |
| 3 T | 4 [65] | 22.5 | 1.05 2.81 | 2.95 | 0.1-0.5 | |
| | | furthe r stirre d coolin g to 10.5 | | | | 1.7 |
| 4 H | 4 [65] | 24.2 | 1.12 2.57 | 2.88 | 2.5-3 | 5.6 |
| C.1 H | 6 [68] | 10.5 | 0.66 8.09 | 5.34 | 2.5-3 | 34.0 |
| 5 H | 4 [64] | 34.2 | 1.66 1.41 | 2.34 | 2.5-3 | 5.7 |
| C.2 H | 3 [62] | 35.0 | 1.72 0.74 | 1.28 | 2.5-3 | 31.0 |
| 6 H | 6 [68] | 35.0 | 1.72 2.49 | 4.28 | 2.5-3 | 1.2 |
| 7 H | 3 [62] | 24.5 | 1.14 1.63 | 1.86 | 2.5-3 | 5.3 |
| 8 H | 6 [68] | 25.5 | 1.18 4.08 | 4.82 | 2.5-3 | 4.6 |
| 9 H | 6 [66] $H_2O$/ MeOH | 22.5 | 1.20 4.00 note[4)] | 4.80 | 0.1-0.5 | 2.8 |

[1)] T/H refers to the water-immiscible solvent used in the column:
T = toluene; H = n-heptane.
Experiment 9 (APM solution in water/methanol) was performed in a column having a diameter of 8 cm. The flow-rate of the feed solution was 1.15 kg/hour in expt.9.

[2)] All experiments, except exp.9, were performed using as feed APM solutions in pure water. In experiment 9 a mixed water / methanol solvent was used (1480 g water and 400 g methanol).

[3)] All values, except for comp.expt.1 and for expt.9, were determined at a pressure difference of 0.25 bar (25 kPa). For comp.expt.1 and expt.9 determination occurred at a pressure difference of 1 bar (100 kPa), but the value shown in the table re-calculated to the value for 0.25 bar (taking the compressibility at 0.57).

[4)] determined on the basis of data from EP-A-0128694

In the table the first column shows the number of the Experiment or Comparative experiment (C.), and also indicates the water-immiscible solvent used: T (=toluene) or H (= n-heptane)). The second column relates to the feed solution and shows the APM-content (in wt.%) and temperature (in °C). It is noticed that Expt.9 has been performed using water / methanol as solvent, whereas all other experiments used pure water as solvent. Further, in experiment 3, the slurry obtained was further cooled with agitation to 10.5°C before cake resistance was determined. The temperature of the slurries obtained is shown in the third column. Column 4 shows the pertaining data for c*, Δc and σ; σ follows from the formula σ = Δc/c*. Column 5 shows the droplet size (diameter) in mm, and column 6 presents the specific cake resistance values at 25 kPa (for instance, "1.9" means "1.9*10[9] m/kg")

From these experiments it can be seen that according to the method of the invention aspartame crystals with good properties are being obtained in a process with efficient and rapid cooling. Comparative experiments 1 and 2, which were performed at an initial relative supersaturation of 8.09 and of 0.74, respectively, clearly yield poor results in specific cake resistance values.

In addition applicant has found that the products obtained in the above experiments according to the invention, after filtering, could be easily handled in a wet granulating process. Achieving equally favorable results in wet granulation is impossible for aspartame crystallized by static crystallization according to EP-A-91787.

Wet granulation tests were performed in an Eirich granulator, consisting of a 5 dm$^3$ rotating vessel positioned at an angle of 30° with the vertical, and having an internal rotor rotating in opposite direction. In the experiments the vessel was rotated at a rotation speed of 42 rpm, while the tip speed of the rotor was chosen at 2.8 m/sec. The water content of the starting product for wet granulation was about 60%, and during the granulation process this water content was lowered to about 30% by passing a stream of air of 65°C over the product layer. Actual granulation started when the water content of the product was about 45- 40 wt.% or lower. Particle size distribution, bulk density and friability of the granular products obtained were determined, as well as their potential for mechanical compaction. Friability is a measure for resistance against particle attrition; higher values of friability show less resistance, i.e higher formation of dust during testing for friability. Such tests can be performed e.g. in rotary drums, optionally with the aid of solid bodies moving within the drum. It was found that aspartame crystals made by the method of the invention could be granulated in about 4-5 hours to obtain granular products having properties as shown below, indicating in square brackets properties of granular products analogously made[1] from aspartame crystallized by the '787 method:

* Particle size distribution (psd; in mm):

$d_{10}$ 0.6-0.8[<0.02]
$d_{50}$ 2.0-3.0[ 0.05]
$d_{90}$ 3.0-8.9[ 0.56]

* Friability (in %; should be < 20): 6-10[60]
* Max. tablet density (in kg/m$^3$):1000-1080[1120] at a max. pressure of (in kN/cm$^2$):9-11[10] with max. tablet strength (in N/cm$^2$):330-420[170]

## Claims

1. Method for cooling an aqueous solution of α-L-aspartyl-L-phenylalanine methyl ester and crystallization of α-L-aspartyl-L-phenylalanine methyl ester therefrom with avoiding of turbulence in the aqueous crystallizing system, which comprises (i) feeding a hot aqueous solution of α-L-aspartyl-L-phenylalanine methyl ester to means for dispersing droplets of said solution; and (ii) dispersing said droplets into a water-immiscible liquid having a temperature at least about 20°C lower than said hot solution, and having such a temperature difference with the hot solution that no nucleation will occur in the droplets while these are passing the water-immiscible liquid, the water-immiscible liquid having a density smaller than the density of the aqueous solution at the same temperature, and having a viscosity of less than 10 mPa.s; and (iii) cooling said water-immiscible liquid so that said droplets are effectively cooled to reach an initial relative supersaturation of α-L-aspartyl-L-phenylalanine methyl ester within the droplet in the range of 1 to 6, preferably 1.2 to 4; and (iv) collecting the cooled droplets after having passed the water-immiscible liquid to crystallize α-L-aspartyl-L-phenylalanine methyl ester without any mechanical agitation; (v) and providing sufficient time for crystallizing α-L-aspartyl-L-phenylalanine methyl ester from the collected aqueous phase.

2. Method according to claim 1, wherein the means for dispersing droplets are placed near the upper liquid level of the water-immiscible liquid.

3. Method according to any of claims 1-2, wherein the means for dispersing droplets are placed at a short distance above the upper liquid level of the water-immiscible liquid, without being in direct contact with said liquid.

4. Method according to any of claims 1-3, wherein said means for dispersing can be moved vertically, and are being adjusted in height during the process according to the position of the upper liquid level.

5. Method according to any of claims 1-4, wherein the dispersing takes place at least 10 cm above the lower level of the water-immiscible liquid.

[1] It is noticed that the moisture content at the start of this experiment was 32%, and wet granulation was possible without adding additional water; due to the lower water content granulation was faster, about 2 hours, and final moisture content was about 23%.

6. Method according to any of claims 1-5, wherein the hot aqueous solution of α-L-aspartyl-L-phenylalanine methyl ester has a temperature of at least 50°C and a concentration of α-L-aspartyl-L-phenylalanine methyl ester of at least 2.5 % by weight.

7. Method according to any of claims 1-6, wherein the cooling of the water-immiscible liquid takes place by indirect cooling.

8. Method according to any of claims 1-7, wherein part of the water-immiscible liquid is circulated, and externally cooled, by withdrawing it from the top of the water-immiscible liquid layer, without entrainment of aqueous phase, and feeding it countercurrently near the lower level of said liquid.

9. Method according to any of claims 1-8, wherein the water-immiscible liquid is contained in (one or more) tubular column(s).

10. Method according to claim 9, wherein the dispersion of droplets occurs at such distance from the internal side-walls of the tubular column(s) that the droplets formed will not come into contact with said side-walls while moving down.

11. Method according to any of claims 1-10, wherein the diameter of the droplets of a-L-aspartyl-L-phenylalanine methyl ester solution formed in the dispersion means is in the range of 0.05 to 5 mm, preferably 0.1 to 3 mm, on average.

12. Method according to any of claims 1-11, wherein the water-immiscible liquid is chosen from the group of aliphatic and/or aromatic hydrocarbons having from 5 to 12 carbon atoms.

13. Method according to claim 12, wherein the water-immiscible liquid is toluene or n-heptane.

14. Method according to any of claims 1-13, wherein the droplets while passing the water-immiscible liquid are cooled to a temperature in the range of 35 to 20°C or lower.

15. Method according to any of claims 1-14, wherein the collected aqueous phase is further cooled by indirect cooling.

16. Method according to any of claims 1-15, wherein the collected aqueous phase is kept without mechanical agitation for at least 30 minutes.

17. Method according to claim 16, wherein the bottom part of the collected aqueous phase is treated to carefully disturb the network of crystals which has formed.

18. Method according to any of claims 1-17, wherein the process is being operated continuously in that amounts of α-L-aspartyl-L-phenylalanine methyl ester crystal slurry are withdrawn from the bottom part of the collected aqueous phase, either continuously or intermittently, after having remained without mechanical agitation for at least 30 minutes.

19. Method according to claim 18, wherein the crystal slurry withdrawn is further cooled in an additional vessel, with our without mechanical agitation, to a temperature in the range of 0-20°C.

20. Method according to any of claims 1-19, wherein - after crystallization of α-L-aspartyl-L-phenylalanine methyl ester - the collected aqueous phase and the water-immiscible liquid are separated, and any water-immiscible liquid remaining in the aqueous phase is removed by a method known per se, such as evaporation.

21. Method and apparatus for cooling an aqueous solution of α-L-aspartyl-L-phenylalanine methyl ester and crystallization of α-L-aspartyl-L-phenylalanine methyl ester therefrom as substantially described in the description and examples.

**Patentansprüche**

1. Verfahren zum Kühlen einer wässerigen Lösung von α-L-Aspartyl-L-phenylalanin-methylester und Kristallisation von α-L-Aspartyl-L-phenylalanin-methylester daraus, mit Vermeiden von Turbulenz in dem wässerigen Kristallisationssystem, welches umfaßt: i) Zuführen einer heißen wässerigen Lösung von α-L-Aspartyl-L-phenylalanin-methylester zu Mitteln zum Dispergieren von Tröpfchen der besagten Lösung; und ii) Dispergieren besagter Tröpfchen in eine mit Wasser nicht mischbare Flüssigkeit mit einer Temperatur von mindestens etwa 20°C niedriger, als die der besagten heißen Lösung und mit solch einem Temperaturunterschied zu der heißem Lösung, dass in den Tröpfchen keine Keimbildung stattfinden wird, während diese die mit Wasser nicht mischbare Flüssigkeit passieren, wobei die mit Wasser nicht mischbare Flüssigkeit eine kleinere Dichte hat, als die Dichte der wässerigen Lösung bei der gleichen Temperatur und eine Viskosität von weniger als 10 mPa.s aufweist; und iii) Kühlen besagter mit Wasser nicht mischbarer Flüssigkeit, so dass besagte Tröpfchen wirksam gekühlt werden, um eine relative Anfangsübersättigung von α-L-Aspartyl-L-phenylalanin-methylester innerhalb der Tröpfchen im Bereich von 1 bis 6, vorzugsweise 1,2 bis 4 zu erreichen; und iv) Sammeln der gekühlten Tröpfchen, nachdem sie die mit Wasser nicht mischbare Flüssigkeit passiert haben, um a-L-Aspartyl-L-phenylalanin-methylester ohne jede mechanische Bewegung zu kristallisieren; v) und Vorsehen von ausreichender Zeit zum Kristallisieren von α-L-Aspartyl-L-phenylalanin-methylester aus der gesammelten wässerigen Phase.

2. Verfahren gemäß Anspruch 1, wobei die Mittel zum Dispergieren von Tröpfchen nahe dem oberen Flüssigkeitsstand der mit Wasser nicht mischbaren Flüssigkeit plaziert werden.

3. Verfahren gemäß einem der Ansprüche 1-2, wobei die Mittel zum Dispergieren von Tröpfchen in einem kurzen Abstand oberhalb des oberen Flüssigkeitsstands der mit Wasser nicht mischbaren Flüssigkeit plaziert werden, ohne in direktem Kontakt mit besagter Flüssigkeit zu sein.

4. Verfahren gemäß einem der Ansprüche 1-3, wobei die Mittel zum Dispergieren vertikal bewegt werden können und während des Verfahrens gemäß der Position des oberen Flüssigkeitsstands in der Höhe angepaßt werden.

5. Verfahren gemäß einem der Ansprüche 1-4, wobei das Dispergieren mindestens 10 cm oberhalb des unteren Stands der mit Wasser nicht mischbaren Flüssigkeit stattfindet.

6. Verfahren gemäß einem der Ansprüche 1-5, wobei die heisse wässerige Lösung von α-L-Aspartyl-L-phenylalanin-methylester eine Temperatur von mindestens 50°C und eine Konzentration von a-L-Aspartyl-L-phenylalanin-methylester von mindestens 2,5 Gew.-% aufweist.

7. Verfahren gemäß einem der Ansprüche 1-6, wobei das Kühlen der mit Wasser nicht mischbaren Flüssigkeit durch indirektes Kühlen stattfindet.

8. Verfahren gemäß einem der Ansprüche 1-7, wobei ein Teil der mit Wassern nicht mischbaren Flüssigkeit durch seine Entnahme oben aus der mit Wasser nicht mischbaren Flüssigkeitsschicht ohne Mitreißen von wässeriger Phase und sein gegenläufiges Zuführen nahe dem unteren Stand der besagten Flüssigkeit zirkuliert und extern gekühlt wird.

9. Verfahren gemäß einem der Ansprüche 1-8, wobei die mit Wasser nicht mischbare Flüssigkeit in (einer oder mehreren) röhrenförmigen Säule(n) enthalten ist.

10. Verfahren gemäß Anspruch 9, wobei die Dispersion von Tröpfchen in solch einem Abstand zu den inneren Seitenwänden der röhrenförmigen Säule(n) stattfindet, daß die gebildeten Tröpfchen nicht mit besagten Seitenwänden in Kontakt kommen werden, während sie sich nach unten bewegen.

11. Verfahren gemäß einem der Ansprüche 1-10, wobei der Durchmesser der in den Dispergiermitteln gebildeten Tröpfchen von α-L-Aspartyl-L-phenylalanin-methylesterlösung im Bereich von durchschnittlich 0,05 bis 5 mm, vorzugsweise 0,1 bis 3 mm liegt.

12. Verfahren gemäß einem der Ansprüche 1-11, wobei die mit Wasser nicht mischbare Flüssigkeit ausgewählt wird aus der Gruppe der aliphatischen und/oder aromatischen Kohlenwasserstoffe mit 5 bis 12 Kohlenstoffatomen.

13. Verfahren gemäß Anspruch 12, wobei die mit Wasser nicht mischbare Flüssigkeit Toluol oder n-Heptan ist.

14. Verfahren gemäß einem der Ansprüche 1-13, wobei die Tröpfchen zu einer Temperatur im Bereich von 35 bis 20°C oder niedriger gekühlt werden, während sie die mit Wasser nicht mischbare Flüssigkeit passieren.

**15.** Verfahren gemäß einem der Ansprüche 1-14, wobei die gesammelte wässerige Phase durch indirektes Kühlen weiter gekühlt wird.

**16.** Verfahren gemäß einem der Ansprüche 1-15, wobei die gesammelte wässerige Phase ohne mechanische Bewegung für mindestens 30 Minuten gehalten wird.

**17.** Verfahren gemäß Anspruch 16, wobei der untere Teil der gesammelten wässerigen Phase behandelt wird, um das sich gebildete Netzwerk an Kristallen vorsichtig zu stören.

**18.** Verfahren gemäß einem der Ansprüche 1-17, wobei das Verfahren dadurch kontinuierlich durchgeführt wird, daß Mengen an $\alpha$-L-Aspartyl-L-phenylalanin-methylester-Kristallaufschlämmung aus dem unteren Teil der gesammelten wässerigen Phase entweder kontinuierlich oder intermittierend entnommen werden, nachdem sie ohne mechanische Bewegung für mindestens 30 Minuten verblieben sind.

**19.** Verfahren gemäß Anspruch 18, wobei die entnommene Kristallaufschlämmung in einem zusätzlichen Gefäß mit oder ohne mechanische Bewegung auf eine Temperatur im Bereich von 0-20°C weiter gekühlt wird.

**20.** Verfahren gemäß einem der Ansprüche 1-19, wobei nach Kristallisation von $\alpha$-L-Aspartyl-L-phenylalanin-methylester die gesammelte wässerige Phase und die mit Wasser nicht mischbare Flüssigkeit getrennt werden und jede in der wässerigen Phase verbleibende, mit Wasser nicht mischbare Flüssigkeit, durch ein per se bekanntes Verfahren wie Abdampfung entfernt wird.

**21.** Verfahren und Vorrichtung zum Kühlen einer wässerigen Lösung von $\alpha$-L-Aspartyl-L-phenylalanin-methylester und Kristallisation von $\alpha$-L-Aspartyl-L-phenylalanin-methylester daraus, wie in der Beschreibung und den Beispielen im wesentlichen beschrieben.

**Revendications**

**1.** Procédé de refroidissement d'une solution aqueuse d'ester méthylique d'a-L-aspartyl-L-phénylalanine et de cristallisation d'ester méthylique d'$\alpha$-L-aspartyl-L-phénylalanine issu de celle-ci en évitant la turbulence dans le système de cristallisation aqueux qui consiste à (i) amener une solution aqueuse chaude d'ester méthylique d'$\alpha$-L-aspartyl-L-phénylalanine à des moyens de dispersion de gouttelettes de ladite solution et (ii) disperser lesdites gouttelettes dans un liquide non miscible dans l'eau ayant une température d'au moins environ 20°C inférieure à celle de ladite solution chaude et ayant une différence de température avec la solution chaude telle qu'aucune nucléation n'a lieu dans les gouttelettes tandis que celles-ci passent dans le liquide non miscible dans l'eau, le liquide non miscible dans l'eau ayant une densité plus petite que la densité de la solution aqueuse à la même température et ayant une viscosité inférieure à 10 mPa.s ; et (iii) refroidir ledit liquide non miscible dans l'eau de sorte que lesdites gouttelettes sont refroidies efficacement pour atteindre une super-saturation relative initiale d'ester méthylique d'$\alpha$-L-aspartyl-L-phénylalanine dans la gouttelette dans la gamme de 1 à 6, de préférence 1,2 à 4 ; et (iv) recueillir les gouttelettes refroidies qui sont passées dans le liquide non miscible dans l'eau pour cristalliser l'ester méthylique d'$\alpha$-L-aspartyl-L-phénylalanine sans agitation mécanique , (v) et fournir un temps suffisant pour cristalliser l'ester méthylique d'$\alpha$-L-aspartyl-L-phénylalanine à partir de la phase aqueuse recueillie.

**2.** Procédé selon la revendication 1, dans lequel les moyens de dispersion des gouttelettes sont placés près du niveau de liquide supérieur du liquide non miscible dans l'eau.

**3.** Procédé selon l'une quelconque des revendications 1 ou 2, dans lequel les moyens de dispersion des gouttelettes sont placés à une courte distance au dessus du niveau de liquide supérieur du liquide non miscible dans l'eau, sans être en contact direct avec ledit liquide.

**4.** Procédé selon l'une quelconque des revendications 1 à 3, dans lequel lesdits moyens de dispersion peuvent se déplacer verticalement et sont réglés en hauteur pendant le procédé selon la position du niveau de liquide supérieur.

**5.** Procédé selon l'une quelconque des revendications 1 à 4, dans lequel la dispersion a lieu au moins 10 cm au dessus du niveau inférieur du liquide non miscible dans l'eau.

**6.** Procédé selon l'une quelconque des revendications 1 à 5, dans lequel la solution aqueuse chaude d'ester méthylique d'α-L-aspartyl-L-phénylalanine a une température d'au moins 50°C et une concentration d'ester méthylique d'α-L-aspartyl-L-phénylalanine d'au moins 2,5% en poids.

**7.** Procédé selon l'une quelconque des revendications 1 à 6, dans lequel le refroidissement du liquide non miscible dans l'eau a lieu par refroidissement indirect.

**8.** Procédé selon l'une quelconque des revendications 1 à 7, dans lequel une partie du liquide non miscible dans l'eau est mise en circulation et refroidie par voie externe en la soutirant du sommet de la couche liquide non miscible dans l'eau sans entraînement de la phase aqueuse et en l'amenant à contre-courant près du niveau inférieur dudit liquide.

**9.** Procédé selon l'une quelconque des revendications 1 à 8, dans lequel le liquide non miscible dans l'eau est contenu dans une ou plusieurs colonnes tubulaires.

**10.** Procédé selon la revendication 9, dans lequel la dispersion des gouttelettes a lieu à une distance telle à partir des parois latérales internes de(s) colonne(s) tubulaire(s) que les gouttelettes formées ne viennent pas en contact avec lesdites parois latérales pendant le déplacement vers le bas.

**11.** Procédé selon l'une quelconque des revendications 1 à 10, dans lequel le diamètre des gouttelettes de la solution d'ester méthylique d'α-L-aspartyl-L-phénylalanine formée dans les moyens de dispersion est dans la gamme de 0,05 à 5 mm, de préférence 0,1 à 3 mm en moyenne.

**12.** Procédé selon l'une quelconque des revendications 1 à 11, dans lequel on choisit le liquide non miscible dans l'eau parmi les hydrocarbures aliphatiques et/ou aromatiques ayant de 5 à 12 atomes de carbone.

**13.** Procédé selon la revendication 12, dans lequel le liquide non miscible dans l'eau est le toluène ou le n-heptane.

**14.** Procédé selon l'une quelconque des revendications 1 à 13, dans lequel les gouttelettes lors du passage dans le liquide non miscible dans l'eau sont refroidies à une température dans la gamme de 35 à 20°C ou moins.

**15.** Procédé selon l'une quelconque des revendications 1 à 14, dans lequel la phase aqueuse recueillie est encore refroidie par refroidissement indirect.

**16.** Procédé selon l'une quelconque des revendications 1 à 15, dans lequel la phase aqueuse recueillie est maintenue sans agitation mécanique pendant au moins 30 minutes.

**17.** Procédé selon la revendication 16, dans lequel le fond de la phase aqueuse recueillie est traité pour perturber soigneusement le réseau de cristaux qui se forme.

**18.** Procédé selon l'une quelconque des revendications 1 à 17, dans lequel le procédé fonctionne en continu en ce que des quantités de bouillie de cristaux d'ester méthylique d'α-L-aspartyl-L-phénylalanine sont soutirées du fond de la phase aqueuse recueillie, soit en continu soit par intermittence, après être restées sans agitation mécanique pendant au moins 30 minutes.

**19.** Procédé selon la revendication 18, dans lequel la bouillie de cristaux soutirée est refroidie encore dans un récipient supplémentaire avec ou sans agitation mécanique à une température dans la gamme de 0 à 20°C.

**20.** Procédé selon l'une quelconque des revendications 1 à 19, dans lequel après cristallisation de l'ester méthylique d'α-L-aspartyl-L-phénylalanine, la phase aqueuse recueillie et le liquide non miscible dans l'eau sont séparés et tout liquide non miscible dans l'eau restant dans la phase aqueuse est enlevé par un procédé connu comme l'évaporation.

**21.** Procédé et appareil pour refroidir une solution aqueuse d'ester méthylique d'α-L-aspartyl-L-phénylalanine et pour la cristallisation de l'ester méthylique d'α-L-aspartyl-L-phénylalanine qui en est issu comme sensiblement décrit dans la description et les exemples.